# EUROPEAN PATENT SPECIFICATION

(11) **EP 1 654 990 B1**
(45) Date of publication and mention of the grant of the patent: **15.08.2007**
(21) Application number: 04026483.0
(22) Date of filing: 08.11.2004
(51) Int. Cl.: A61B 17/11

(54) **Pipe clip for securing an anastomosis**
Rohrklemme zur Haltung einer Anastomose
Collier de serrage pour la fixation d'une anastomose

(43) Date of publication of application: 10.05.2006
(73) Proprietor: Inderbitzi, Rolf, Dr. med., 8967 Widen (CH)
(72) Inventor: Inderbitzi, Rolf, Dr. med., 8967 Widen (CH)
(74) Representative: Heinze, Ekkehard

(56) References cited:
- US-A- 3 435 823
- US-A- 5 741 274
- US-A- 5 843 170
- US-A1- 2004 186 548
- US-B1- 6 248 116

## Description

### BACKGROUND

The present invention relates to the field of vascular surgery. In particular, the present invention provides a clip for securing an anastomosis between two blood vessels, or between a blood vessel and a vascular prosthesis. More particularly, the present invention can be used for securing an end-to-end anastomosis between the abdominal aorta and a vascular prosthesis.

An anastomosis is a surgical connection between two structures, which is most commonly created between tubular structures, such as between blood vessels or loops of the intestine. Anastomosis between blood vessels is indicated for the repair of a diseased or damaged section of a blood vessel.

An anastomosis is commonly achieved by stitching the structures together with sutures. However, the procedure of suturing is time-consuming. Moreover, suturing inflicts additional trauma on the structures. This may prolong the healing process, compromise the results of the surgery, or lead to delayed problems such as stenosis.

With advancement in technology, anastomoses are being performed by means of sutureless technologies such as stapling devices or tissue adhesives.

The use of stapling devices for the creation of anastomosis between tubular organs such as a blood vessel is traumatic to the blood vessel. When using tissue adhesives, a stay suture has to be used till the adhesive bonds strongly. Moreover, tissue adhesives may not be effective in anastomosis where there is high tension at the anastomotic site, e.g., in cases of anastomosis involving the aorta.

Hence, it is tough to achieve a safe, secure and a leak-proof anastomosis with the use of sutureless techniques such as stapling devices and tissue adhesives.

Anastomoses involving large blood vessels should be secure and leak proof over a long period. Failure to achieve a leak-proof anastomosis may result in internal bleeding, which may be fatal. Moreover, the wall of the anastomosis weakens over a period of time, and this may result in the development of a false aneurysm at the site of the anastomosis.

Document US 5 741 274 discloses a pipe clip comprising a body member which at its turn comprises attachment means to attach the pipe clip to a prosthesis. The pipe clip has at the fixed end of the body member a connector.

Document US 6 248 116 discloses a pipe clip comprising a lattice of wire members embedded in a protective layer to damp the contact with the vessel. The pipe clip disclosed by this document is attached by stitching or by stapling.

In light of the above discussion, there is a need for a sutureless system to secure anastomosis between tubular structures such as blood vessels. The system would need to ensure a safe, secure, and a leak-proof anastomosis. Further, the system should prevent the development of a false aneurysm.

### SUMMARY

An object of the invention is to provide a pipe clip suitable for securing an anastomosis by using a sutureless technique.

Another object of the present invention is to provide a system for securing an anastomosis by using a sutureless technique.

Yet another object of the present invention is to provide a traction device for fixing the pipe clip at the site of the anastomosis.

In accordance with an exemplary embodiment of the present invention, a pipe clip and a traction device are provided for use in securing an anastomosis. The pipe clip comprises a body member, a connector and traction means. The body member has a wire lattice with longitudinal and transverse wire members embedded in it. The longitudinal wire members and transverse wire members have attachment means that have a straight part and a pointed tip. The pointed tip helps to penetrate the surface of the structures that form the anastomosis, and enables a firm fix between the pipe clip and the site of the anastomosis.

In accordance with an exemplary embodiment of the present invention, the traction device comprises a handgrip, a body, a traction hook, and a longitudinal member. The traction device helps in fixing the pipe clip at the site of the anastomosis.

A method is presented for securing an anastomosis, using a pipe clip. After the anastomosis has been secured, the pipe clip is placed around the site of the anastomosis. The pipe clip is fixed at the site of the anastomosis, using the traction device.

The additional objects and advantages of the invention will become apparent to those skilled in the art, by referring to the following detailed description, in conjunction with the figures provided.

### BRIEF DESCRIPTION OF THE DRAWINGS

The preferred embodiments of the invention will hereinafter be described in conjunction with the appended drawings provided to illustrate and not to limit the invention, wherein like designations denote like elements, and in which:
FIG. 1 is a representation of a pipe clip (100), in accordance with an embodiment of the present invention;
FIG. 2 is a representation of a wire lattice (200), in accordance with an embodiment of the present invention;
FIG. 3 is a representation of attachment means (206) embedded in a body member, in accordance with an embodiment of the present invention;
FIG. 4 is a representation of a cross section of the body member (102), in accordance with an embodiment of the present invention;
FIG. 5 is a representation of a connector (108), in accordance with an embodiment of the present invention;
FIG. 6 is a representation of the pipe clip (100) in a closed position, in accordance with an embodiment of the present invention;
FIG. 7 is a representation of a traction device (700), in accordance with an embodiment of the present invention;
FIG. 8 is a flowchart of a method for securing an anastomosis, in accordance with an embodiment of the present invention;
FIG. 9 is a flowchart of a method for placing the pipe clip at the site of the anastomosis, in accordance with an embodiment of the present invention;
FIG. 10 is a flowchart of a method for fixing a pipe clip at the site of the anastomosis, in accordance with an embodiment of the present invention;
FIG. 11 is a representation of the pipe clip, wherein the pipe clip has been fixed around an anastomosis between the abdominal aorta and an in-out-lay-prosthesis (IOLP), in accordance with an embodiment of the present invention; and
Fig. 12 is a representation of the IOLP.

### DETAILED DESCRIPTION OF THE INVENTION

The present invention discloses a pipe clip for securing an anastomosis without the use of sutures, and a traction device for fixing the pipe clip at the site of the anastomosis. In particular, the present invention discloses a pipe clip for securing an anastomosis between the abdominal aorta and a vascular prosthesis. The pipe clip is placed around the site of the anastomosis and fixed to it with the help of the traction device. The application of the pipe clip leads to the formation of a secure and leak-proof anastomosis between the abdominal aorta and the vascular prosthesis, without the use of sutures. Further, the pipe clip provides external support at the site of the anastomosis and prevents the development of a false aneurysm.

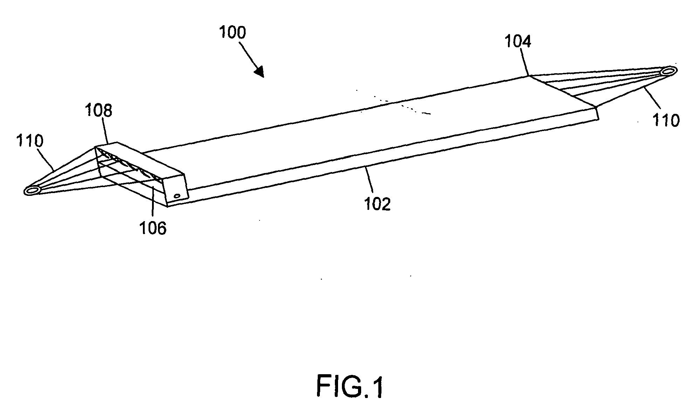

FIG. 1 is a representation of a pipe clip 100, in accordance with an embodiment of the present invention. Pipe clip 100 has a body member 102. Body member 102 has a free end 104 and a fixed end 106. A connector 108 is attached to fixed end 106 of body member 102. In accordance with an embodiment of the present invention, traction means 110 are attached to free end 104 of body member 102 and connector 108.

The circumference of the anastomosis site can be determined preoperatively by using imaging modalities such as computed tomography. The length of body member 102 should be within a range of 10 mm to 25 mm, preferably 15 mm more than the determined circumference of the site of the anastomosis. A pipe clip 100, with a body member 102 of the appropriate length, can therefore be selected preoperatively.

In accordance with an embodiment of the present invention, the height of body member 102 is within a range of 2.2 mm to 3.2 mm, the preferred height being 2.7 mm. In accordance with an embodiment of the present invention, the width of body member 102 is within a range of 9 mm to 21 mm, the preferred width being 15 mm.

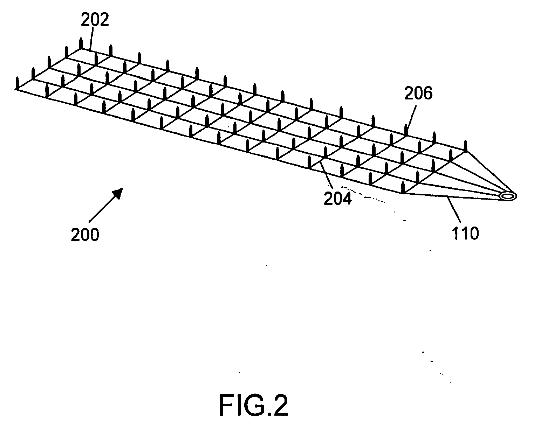

FIG. 2 is a representation of a wire lattice 200, in accordance with an embodiment of the present invention. Wire lattice 200 is embedded in body member 102. In accordance with an embodiment of the present invention, wire lattice 200 has longitudinal wire members 202 and transverse wire members 204. Attachment means 206 are attached to longitudinal wire members 202 and transverse wire members 204. In accordance with an embodiment of the present invention, traction means 110 is attached to wire lattice 200 at free end 104 of body 102.

In accordance with an embodiment of the present invention, the length of longitudinal wire members 202 is equal to the length of body member 102. In accordance with an embodiment of the present invention, the length of transverse wire members 204 is within a range of 9 mm to 24 mm, the preferred length being 14 mm.

In accordance with an embodiment of the present invention, the diameter of longitudinal wire members 202 and transverse wire members 204 is within a range of 0.5 mm to 1.5 mm, the preferred diameter being 1.0 mm. In accordance with an embodiment of the present invention, longitudinal wire members 202 and transverse wire members 204 are made of a biocompatible material. In accordance with an embodiment of the present invention, the biocompatible material is titan.

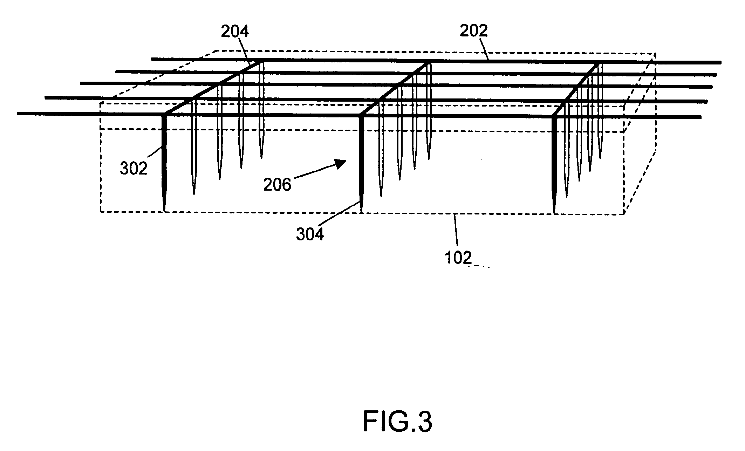

FIG. 3 is a representation of attachment means 206, in accordance with an embodiment of the present invention. Attachment means 206 has a straight part 302 and a pointed tip 304. On closing pipe clip 100 around the site of the anastomosis, pointed tip 304 penetrates the structures that form the anastomosis and helps in firmly fixing pipe clip 100, thereby securing the site of the anastomosis.

In accordance with an embodiment of the present invention, the length of attachment means 206 is within a range of 2 mm to 3 mm, the preferred length being 2.5 mm. In accordance with an embodiment of the present invention, attachment means 206 are made of biocompatible material. In accordance with an embodiment of the present invention, the biocompatible material is titan.

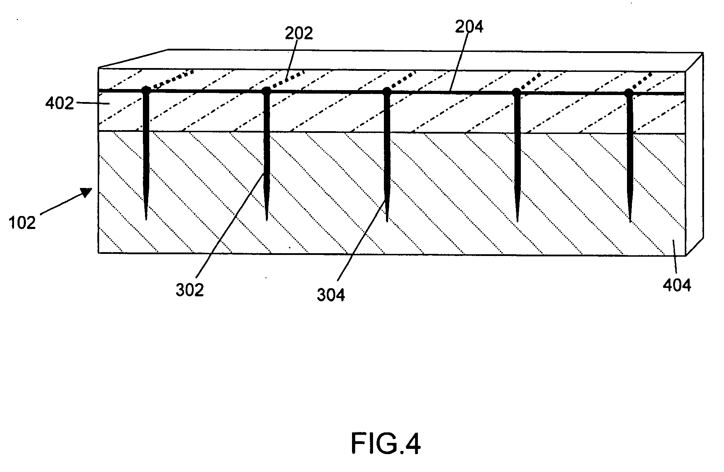

FIG. 4 is a cross sectional view of body member 102, in accordance with an embodiment of the present invention. Body member 102 has a compact layer 402 and a compressible layer 404. Longitudinal wire members 202 and transverse wire members 204 are embedded in compact layer 402. Straight part 302 of attachment means 206 is embedded in compact layer 402 and compressible layer 404.

In accordance with an embodiment of the present invention, compact layer 402 is made of a non-compressible biocompatible material. In accordance with an embodiment of the present invention, the non-compressible biocompatible material is Poly Tetra Fluoro Ethylene (PTFE). In accordance with an embodiment of the present invention, compressible layer 404 is made of a compressible biocompatible material. In accordance with an embodiment of the present invention, the compressible biocompatible material is PTFE sponge. In accordance with an embodiment of the present invention, the thickness of compact layer 402 is within a range of 0.1 mm to 1.5 mm, the preferred thickness being 0.2 mm. In accordance with an embodiment of the present invention, the thickness of compressible layer 404 is within a range of 1 to 3 mm, the preferred thickness being 2.5 mm.

Compact layer 402 helps in firmly fixing longitudinal wire members 202 and transverse wire members 204 to the body, and, along with wire members 202, forms the backbone of pipe clip 100. Upon fastening pipe clip 100 around the site of the anastomosis, compressible layer 404 is compressed and attachment means 206 penetrate the surface of compressible layer 404. After penetrating the surface of compressible layer 404, attachment means 206 penetrate the structures forming the anastomosis, thereby fixing pipe clip 100 at the site of the anastomosis.

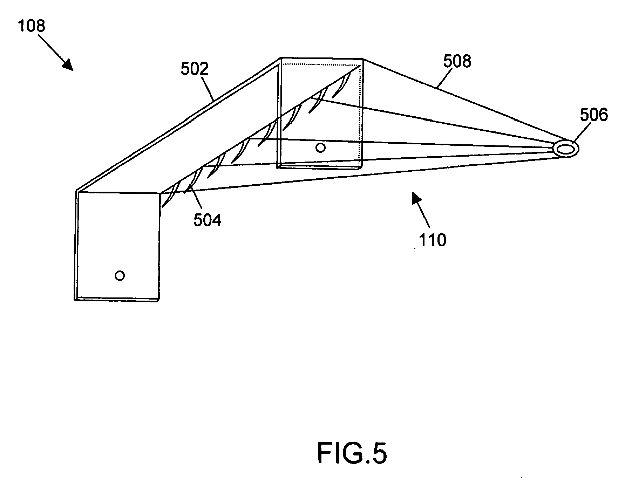

FIG. 5 is a representation of connector 108, in accordance with an embodiment of the present invention. Connector 108 has a rectangular member 502 and projections 504. Traction means 110 is attached to rectangular member 502. In accordance with an embodiment of the present invention, traction means 110 has a loop 506 and wires 508 connecting loop 506 with rectangular member 502.

Rectangular member 502 and fixed end 106 form an opening through which free end 104 is threaded. Rectangular member 502 helps to hold free end 104 over fixed end 106.

Projections 504 help in fixing free end 104 of body member 102 to rectangular member 502. Projections 504 prevent the slipping of free end 104, thereby fixing free end 104 to rectangular member 502.

In accordance with an embodiment of the present invention, the height of rectangular member 502 is within a range of 4.5 mm to 9 mm, the preferred height being 7 mm. In accordance with an embodiment of the present invention, the length of the projections 504 is within a range of 1.5 mm to 3.5 mm, the preferred length being 1.5 mm. In accordance with an embodiment of the present invention, the rectangular member is made of a biocompatible material. In accordance with an embodiment of the present invention, the biocompatible material is titan.

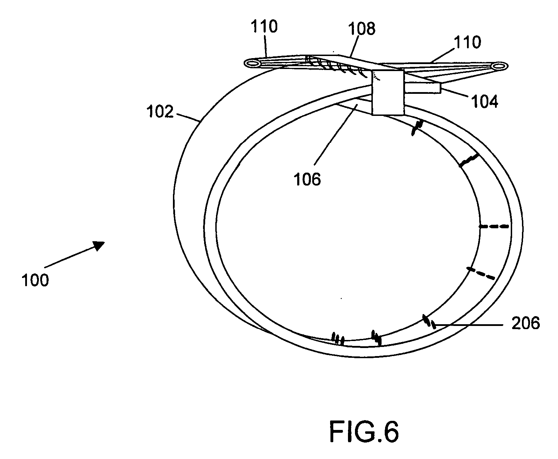

FIG. 6 is a representation of pipe clip 100 in a closed position, in accordance with an embodiment of the present invention. Free end 104 has been joined to fixed end 106 with the help of connector 108. Traction means 110 are attached to free end 104 and connector 108. In the closed position, compressible layer 404 of body member 102 is compressed when traction is applied through traction means 110. Compression of compressible layer 404 results in the penetration of attachment means 206 through its surface.

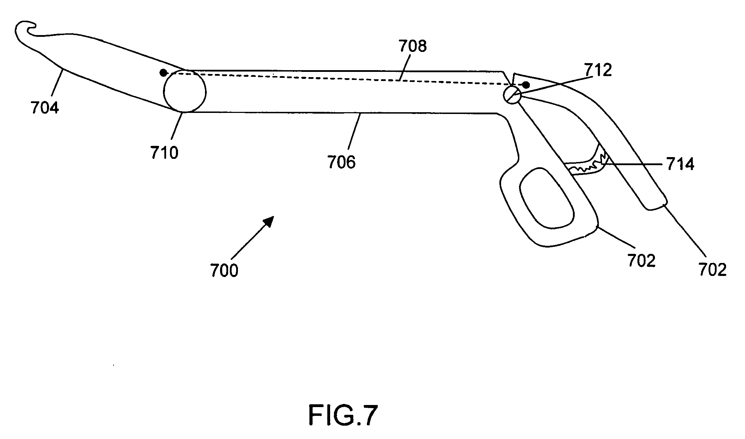

FIG. 7 is a representation of a traction device 700, in accordance with an embodiment of the present invention. Traction device 700 has a handgrip 702, a traction hook 704, a body 706, and a longitudinal member 708. Traction device 700 has a joint 710 between body 706 and traction hook 704. Handgrip 702 has a fulcrum 712 that helps to transfer the force from handgrip 702 to traction hook 704. There is a ratchet 714 to control the movement of handgrip 702. In accordance with an embodiment of the present invention, joint 710 is a hinge joint. Longitudinal member 708 is attached to handgrip 702 at one end and to traction hook 704 at the other end. On applying force to handgrip 702, the force is transmitted by longitudinal member 708 to traction hook 704.

In accordance with an embodiment of the present invention, handgrip 702, traction hook 704, body 706, ratchet 714, and longitudinal member 708 are made of metal alloy. In accordance with an embodiment of the present invention, joint 710 and fulcrum 712 are made from firm silicone.

Having disclosed pipe clip 100 and traction device for fixing pipe clip 100, a method for securing an anastomosis, by using these, is disclosed hereinafter. A procedure for applying pipe clip 100 to an anastomosis between the abdominal aorta and a vascular prosthesis is described below, as an example of securing an anastomosis. It will be apparent to one skilled in the art that the same procedure can also be applied to an anastomosis between other structures.

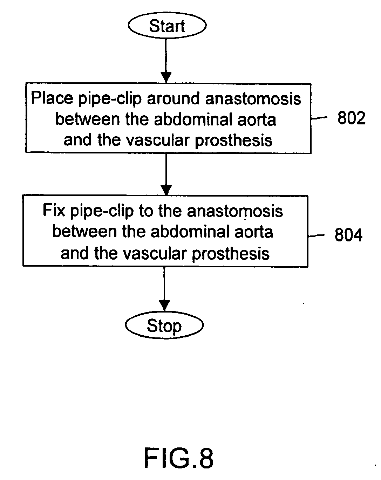

FIG. 8 is a flowchart of a method for securing an anastomosis between the abdominal aorta and a vascular prosthesis. At step 802, pipe clip 100 is placed around the anastomosis between the abdominal aorta and the vascular prosthesis. At step 804, pipe clip 100 is fixed to the site of the anastomosis. Each of the above-mentioned steps is described in detail, hereinafter.

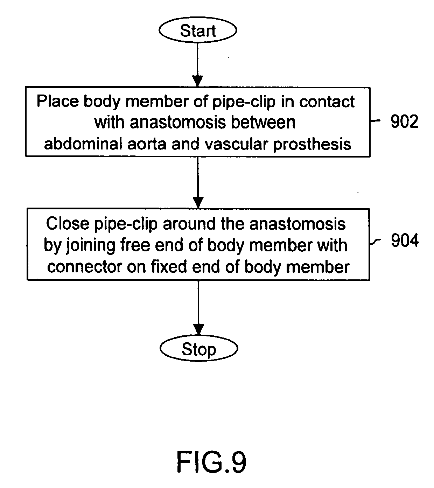

FIG. 9 is a flowchart of a method for placing pipe clip 100 around the anastomosis between the abdominal aorta and the vascular prosthesis. At step 902, body member 102 of pipe clip 100 is placed in contact with the site of the anastomosis. Pipe clip 100 is placed so that compressible layer 404 is in contact with the site of the anastomosis. At step 904, free end 104 is joined with connector 108 by threading free end 104 through the opening between rectangular member 502 and fixed end 106. Projections 504 fix free end 104 to rectangular member 502 and prevent it from slipping, thereby joining free end 104 to connector 108.

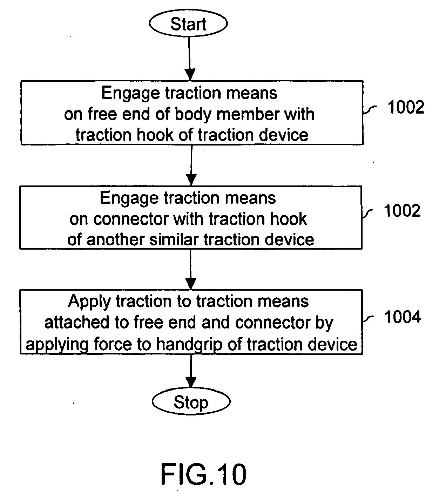

FIG. 10 is a flowchart of a method for fixing pipe clip 100 around the anastomosis between the abdominal aorta and the vascular prosthesis. At step 1002, traction means 110, attached to free end 104, is engaged with traction hook 704 of a traction device 700.
Traction means 110 is engaged with traction hook 504 by threading the tip of traction hook 504 through loop 506 of traction means 110. At step 1004, traction means 110, attached to rectangular member 502 of connector 108, is engaged with traction hook 704 of another similar traction device 700. At step 1006, traction is applied to traction means 110, attached to free end 104 and rectangular member 502 of connector 108. Traction is applied to traction means 110 by applying force to handgrip 702 of traction device 700. Longitudinal member 708 transmits the force applied to handgrip 702 to traction hook 704. As traction hook 704 is engaged with traction means 110, the force is transmitted to traction means 110.

Application of force to traction means 110 leads to the firm fixing of pipe clip 100 around the site of the anastomosis. Firm fixing of pipe clip 100 results in compression of compressible layer 404 of body member 102. On the compression of compressible layer 404, attachment means 206 penetrate the surface of compressible layer 404 and press into the wall of the abdominal aorta as well as the vascular prosthesis, thereby firmly fixing pipe clip 100 around the anastomosis. The pressed compressible layer 404 tightly seals the site of the anastomosis, thereby creating a leak-proof anastomosis and decreasing the chances of any hemorrhage from the anastomosis site.

In accordance with an embodiment of the present invention, pipe clip 100 is used in conjunction with an IOLP, as described hereinafter.

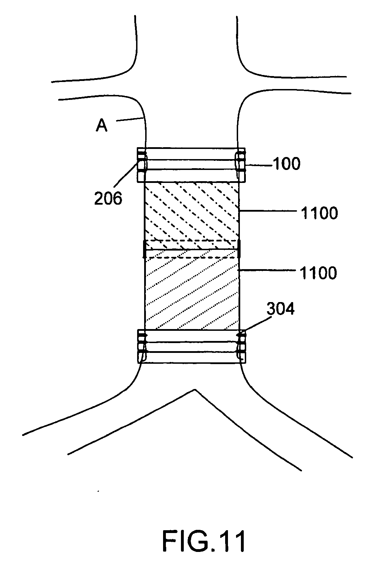

FIG. 11 is a representation of a completed procedure for securing an anastomosis between abdominal aorta A and two similar IOLP 1100. Pipe clip 100 has been applied around the site of the anastomosis, and pointed tip 304 of attachment means 206 are penetrating the wall of the abdominal aorta and IOLP 1100.

Compressible layer 404 is pressed on the site of the anastomosis and makes it leak proof. Further, the application of pipe clip 100 at the site of the anastomosis helps to prevent the development of false aneurysm.

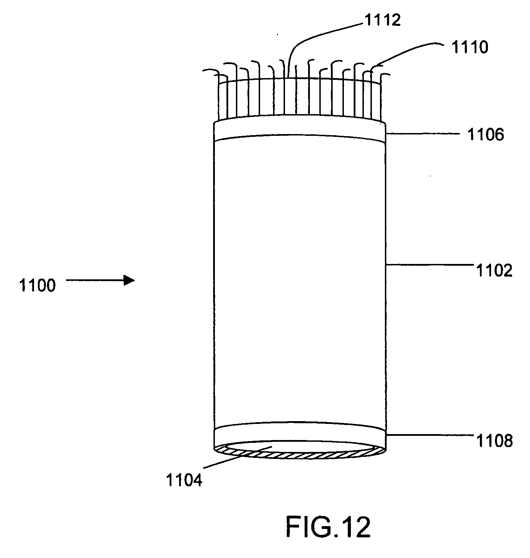

FIG. 12 is a representation of IOLP 1100, which is used for replacing the diseased section of a blood vessel. IOLP 1100 has a tubular body member 1102, a lumen 1104, a fixing end 1106, and a connecting end 1108. Lumen 1104 acts as a conduit for the passage of blood. Attachment means 1110 are present on fixing end 1106, which help in securing IOLP 1100 to the wall of the blood vessel. A membrane 1112 is present on fixing end 1106. Attachment means 1110 are embedded in membrane 1112. Membrane 1112 provides support to attachment means 1110 in order to achieve a secure fixation of IOLP 1100 inside the blood vessel.

The length and the diameter of the diseased section of the blood vessel can be determined pre-operatively by using various diagnostic modalities, such as contrast-enhanced computerized tomography. IOLP 1100 having the appropriate dimensions can thus be chosen pre-operatively.

Attachment means 1110 are of at least two different lengths are attached to fixing end 1106 of IOLP 1100. This arrangement of attachment means of at least two different lengths helps attachment means 1110 to penetrate the wall of the blood vessel at, at least, two different levels. This helps to prevent dislodgement of IOLP 1100 inside the blood vessel.

The thickness of the wall of tubular body member 1102 is in a range of 1 mm to 3 mm, the preferred thickness being 2 mm. Preferably, tubular body member 1102 is made of a biocompatible material. Further preferably, the biocompatible material is polytetrafluoroethylene (PTFE).

Such in-out-lay prosthesis basically comprises a tubular body member acting as a conduit for the passage of blood, the tubular body member comprising at least one fixing end for fixing the in-out-lay prosthesis to the wall of the blood vessel and at least one connecting end for joining one in-out-lay prosthesis with another similar in-out-lay prosthesis, a plurality of attachment means on the fixing end of the tubular body member, the attachment means helping in fixing the in-out-lay prosthesis to the wall of the blood vessel, a membrane on the fixing end of the tubular body member, the membrane helping in supporting the plurality of the attachment means, and a connecting means on the connecting end of the tubular body member, the connecting means helping in joining the in-out-lay prosthesis to another similar in-out-lay prosthesis.

Preferably, the membrane is made of a flexible biocompatible material.

Further preferably, at least one of the plurality of the attachment means comprises a straight part fixing the attachment means to the tubular body member, the straight part being embedded in the membrane, and a hook securing the in-out-lay prosthesis inside the blood vessel, the hook projecting from the membrane.

At least one of the plurality of the attachment means is preferably made of an elastic biocompatible material.

The connecting means preferably comprises a circular latch helping in joining the connecting means of one in-out-lay prosthesis with the connecting means of another similar in-out-lay prosthesis, and a cover flap helping in making the joint between two similar in-out-lay prosthesis leak proof.

While the various embodiments of the invention have been illustrated and described, it will be clear that the invention is not limited to these embodiments only. Numerous modifications, changes, variations, substitutions and equivalents will be apparent to those skilled in the art, without departing from the scope of the invention, as described in the claims.

## Claims

1. A pipe clip (100) for securing an anastomosis, the pipe clip comprising:
a. a body member (102), the body member having a fixed end (106) and a free end (104), the body member comprising:
i. a plurality of wire members (110), wherein the plurality of wire members form a wire lattice (200);
ii. a plurality of attachment means (206), wherein the plurality of attachment means are attached to the plurality of wire members; and
b. a connector (108), wherein the connector is attached to the fixed end of the body member.

2. The pipe clip of claim 1 **characterized in that** at least one of the plurality of attachment means comprises:
a. a straight part (302), wherein the straight part is attached to one of the plurality of wire members; and
b. a tip (304), wherein the tip has a pointed end, the pointed end helping to fix the pipe clip over the site of the anastomosis.

3. The pipe clip of claim 1 or 2, **characterized in that** the connector comprises a plurality of projections (504), the plurality of projections helping to fix the free end of the body member to the connector.

4. The pipe clip of one of the preceding claims, **characterized in that** it further comprises traction means (110), wherein the traction means help to apply force to close the pipe clip at the site of the anastomosis.

5. The pipe clip of claim 4, **characterized in that** at least one of the traction means is attached to the free end of the body member.

6. The pipe clip of claim 4 or 5, **characterized in that** at least one of the traction means is attached to the connector.

7. The pipe clip of one of the preceding claims, **characterized in that** the body member is made of a compressible biocompatible material.

8. A system for securing an anastomosis, the system comprising:
a. a pipe clip (100) according to one of claims 1 to 7.
b. at least one traction device (700), wherein the traction device helps in applying traction to fix the pipe clip at the site of the anastomosis.

9. The system of claim 8, **characterized in that** the traction device comprises:
a. a traction hook (704), wherein the traction hook engages with at least one traction means, the traction hook helping to apply force to the traction means for fixing the pipe clip at the site of the anastomosis;
b. a hand grip (702), wherein the hand grip helps to apply force to the traction hook; and
c. a body (702), wherein the body helps to transmit the force from the handgrip to the traction hook.

## Patentansprüche

1. Gefäßklemme (100) zum sicheren Setzen einer Anastomose, wobei die Gefäßklemme umfasst
a. ein Korpusteil (102), wobei das Korpusteil ein feststehendes Ende (106) und ein freies Ende (104) hat, wobei das Korpusteil umfasst:
i. mehrere Drahtteile (110), wobei die mehreren Drahtteile ein Drahtgitter (200) bilden;
ii. mehrere Befestigungseinrichtungen (206), wobei die mehreren Befestigungseinrichtungen an den mehreren Drahtteilen befestigt sind; und
b. einen Verbinder (108), wobei der Verbinder am feststehenden Ende des Korpusteils befestigt ist.

2. Gefäßklemme nach Anspruch 1, **dadurch gekennzeichnet, dass** mindestens eine der mehreren Befestigungseinrichtungen umfasst:
a. ein gerades Teil (302), wobei das gerade Teil an einem der mehreren Drahtteile befestigt ist; und
b. eine Spitze (304), wobei die Spitze ein zugespitztes Ende hat, wobei das zugespitzte Ende dazu beiträgt, die Gefäßklemme über der Stelle der Anastomose zu fixieren.

3. Gefäßklemme nach Anspruch 1 oder 2, **dadurch gekennzeichnet, dass** der Verbinder mehrere Vorsprünge (504) umfasst, wobei die mehreren Vorsprünge dazu beitragen, das freie Ende des Korpusteils am Verbinder zu fixieren.

4. Gefäßklemme nach einem der vorhergehenden Ansprüche, **dadurch gekennzeichnet, dass** sie darüber hinaus Zugeinrichtungen (110) umfasst, wobei die Zugeinrichtungen dazu beitragen, eine Kraft zum Schließen der Gefäßklemme an der Stelle der Anastomose anzulegen.

5. Gefäßklemme nach Anspruch 4, **dadurch gekennzeichnet, dass** mindestens eine der Zugeinrichtungen am freien Ende des Korpusteils befestigt ist.

6. Gefäßklemme nach Anspruch 4 oder 5, **dadurch gekennzeichnet, dass** mindestens eine der Zugeinrichtungen am Verbinder befestigt ist.

7. Gefäßklemme nach einem der vorhergehenden Ansprüche, **dadurch gekennzeichnet, dass** das Korpusteil aus einem komprimierbaren biokompatiblen Material besteht.

8. System zum sicheren Setzen einer Anastomose, wobei das System umfasst:
a. eine Gefäßklemme (100) nach einem der Ansprüche 1 bis 7;
b. mindestens eine Zugvorrichtung (700), wobei die Zugvorrichtung dazu beiträgt, einen Zug anzulegen, um die Gefäßklemme an der Stelle der Anastomose zu fixieren.

9. System nach Anspruch 8, **dadurch gekennzeichnet, dass** die Zugvorrichtung umfasst:
a. einen Zughaken (704), wobei der Zughaken mit mindestens einer der Zugeinrichtungen ineinander greift, wobei der Zughaken dazu beiträgt, eine Kraft an die Zugeinrichtung anzulegen, um die Gefäßklemme an der Stelle der Anastomose zu fixieren;
b. einen Handgriff (702), wobei der Handgriff dazu beiträgt, eine Kraft an den Zughaken anzulegen; und
c. einen Körper (702), wobei der Körper dazu beiträgt, die Kraft vom Handgriff auf den Zughaken zu übertragen.

## Revendications

1. Clips vasculaire (100) pour fixer une anastomose, le clips vasculaire comprenant :
a. un élément corps (102), l'élément corps possédant une extrémité fixée (106) et une extrémité libre (104), l'élément corps comprenant :
i. de multiples éléments fil (110), les multiples éléments fil formant une grille de fils (200) ;
ii. de multiples moyens de fixation (206), les multiples moyens de fixation étant fixés aux multiples éléments fil ; et
b. un connecteur (108), le connecteur étant fixé à l'extrémité fixée de l'élément corps.

2. Clips vasculaire selon la revendication 1, **caractérisé en ce qu'**au moins l'un des multiples moyens de fixation comprend :
a. une partie droite (302), la partie droite étant attachée à une extrémité des multiples éléments fil ;
b. une pointe (304), la pointe ayant une extrémité pointue, l'extrémité pointue aidant la fixation du clips vasculaire sur l'emplacement de l'anastomose.

3. Clips vasculaire selon la revendication 1 ou 2, **caractérisé en ce que** le connecteur comprend de multiples projections (504), les multiples projections aidant à fixer l'extrémité libre de l'élément corps au connecteur.

4. Clips vasculaire selon l'une quelconque des revendications précédentes, **caractérisé en ce qu'**il comprend en outre des moyens de traction (110), les moyens de traction aidant à appliquer une force pour fermer le clips vasculaire à l'emplacement de l'anastomose.

5. Clips vasculaire selon la revendication 4, **caractérisé en ce qu'**au moins l'un des moyens de traction est fixé à l'extrémité libre de l'élément corps.

6. Clips vasculaire selon la revendication 4 ou 5, **caractérisé en ce qu'**au moins l'un des moyens de traction est fixé au connecteur.

7. Clips vasculaire selon l'une quelconque des revendications précédentes, **caractérisé en ce que** l'élément corps est réalisé en un matériau biocompatible compressible.

8. Système de fixation d'une anastomose, le système comprenant :
a. un clips vasculaire (100) selon l'une quelconque des revendications 1 à 7 ;
b. au moins un dispositif de traction (700), le dispositif de traction aidant à appliquer une traction pour fixer le clips vasculaire à l'emplacement de l'anastomose.

9. Système selon la revendication 8, **caractérisé en ce que** le dispositif de traction comprend :
a. un crochet de traction (704), le crochet de traction s'engageant avec au moins un moyen de traction, le crochet de traction aidant à appliquer une force au moyen de traction pour fixer le clips vasculaire à l'emplacement de l'anastomose ;
b. une poignée (702), la poignée aidant à appliquer une force au crochet de traction ; et
c. un corps (702), le corps aidant à transmettre la force de la poignée au crochet de traction.
